# EUROPÄISCHE PATENTSCHRIFT

(11) **EP 3 708 128 B1**
(45) Veröffentlichungstag und Bekanntmachung des Hinweises auf die Patenterteilung: **01.12.2021**
(21) Anmeldenummer: 20167047.8
(22) Anmeldetag: 08.05.2017
(51) Int. Cl.: A61F 2/80

(54) **PROTHESENSCHAFT UND VERFAHREN ZUR STEUERUNG EINER ANPASSUNG EINES INNENUMFANGES EINES PROTHESENSCHAFTES**
PROSTHESIS SHAFT AND METHOD FOR CONTROLLING AN ADAPTATION OF AN INNER CIRCUMFERENCE OF A PROSTHESIS SHAFT
TIGE DE PROTHÈSE ET PROCÉDÉ DE COMMANDE D'UN RÉGLAGE DE LA PÉRIPHÉRIE INTÉRIEURE D'UNE TIGE DE PROTHÈSE

(30) Priorität: 10.05.2016 DE 102016108631
(43) Veröffentlichungstag der Anmeldung: 16.09.2020
(62) Teilanmeldung aus: 17721723.9
(73) Patentinhaber: Ottobock SE & Co. KGaA, 37115 Duderstadt (DE)
(72) Erfinder: WILL, Christian, 37079 Göttingen (DE); FINKE, Lars Benjamin, 37136 Landolfshausen (DE)
(74) Vertreter: Gramm, Lins & Partner Patent- und Rechtsanwälte PartGmbB

(56) Entgegenhaltungen:
- US-A1- 2010 274 364
- US-A1- 2012 209 404
- US-A1- 2014 121 783
- US-A1- 2015 265 434

## Beschreibung

Die Erfindung betrifft einen Prothesenschaft mit einer proximalen Einführöffnung und einem einen Stumpf zumindest teilweise umgebenden Innenumfang, zumindest einer Anschlussvorrichtung für eine Prothesenkomponente, die an dem Prothesenschaft befestigbar ist und zumindest einem Aktuator, über den der Innenumfang des Prothesenschaftes veränderbar ist. Die Erfindung betrifft ebenfalls ein Verfahren zur Steuerung einer Anpassung eines Innenumfanges eines solchen Prothesenschaftes.

Prothesenschäfte dienen zur Aufnahme von Stümpfen, beispielsweise von Amputationsstümpfen und stellen sicher, dass weitere Prothesenkomponenten sicher an der verbliebenen Gliedmaße gehalten werden. Um ein sicheres Festlegen des Prothesenschaftes an dem Stumpf zu gewährleisten, sind verschiedene Befestigungskonzepte entwickelt worden. Ein Konzept sieht eine im Wesentlichen geschlossene, mit einer Einführöffnung versehene, formstabile Hülle vor, in die der mit einem Prothesenliner versehene Stumpf eingeführt wird. Der Prothesenschaft hält dann über Unterdruck an dem Liner.

Eine weitere Möglichkeit besteht darin, einen Prothesenliner mit einer mechanischen Verriegelungseinrichtung auszustatten, beispielsweise einem Stift, der in eine Aufnahme innerhalb des Prothesenschaftes eingeführt und dort formschlüssig verriegelt wird. Beiden Befestigungskonzepten ist gemeinsam, dass die Innenkontur des Prothesenschaftes möglichst genau der Außenkontur des Stumpfes mit einer Zugabe für den Liner entsprechen sollte. Um dies zu erreichen, wird ein Stumpfabdruck genommen, ein Positivmodel des Stumpfes angefertigt und anhand dieses Models ein individueller Prothesenschaft aus in der Regel faserverstärktem Kunststoff gefertigt.

Neben solch in Umfangsrichtung geschlossenen Schäften sind Prothesenschäfte bekannt, die in Radialrichtung verstellbar ausgeführt sind. Die DE 10 2010 019 843 A1 betrifft einen Prothesenschaft mit einem distalen Endstück und Befestigungseinrichtungen für ein Prothesenkniegelenk und einer radial verspannbaren Hülse sowie einer Spanneinrichtung zum Anpassen eines Aufnahmeraumes der Hülse an den Stumpf. Die Hülse kann zumindest zwei Segmente aufweisen, die mit einem Stützrahmen verbunden sind und einander abschnittsweise überlappen. Die Spanneinrichtung hat zumindest einen Seilzug, der die Segmente überstreckt und mittels einer Stellvorrichtung in der Wirklänge verstellbar ist.

Die DE 10 2006 046 928 A1 betrifft einen Prothesenschaft mit einem flexiblen Innenschaft und einem stabilen Außenschaft, der ein distales Endstück aufweist, von dem aus sich nach proximal erstreckende, zwischen sich einen Zwischenraum ausbildende Schalensegmente erstrecken. Die Schalensegmente können gegen einen elastischen Widerstand aus einer Ausgangsposition schwenkbar mit dem distalen Endstück verbunden sein. Zwischen den Schalensegmenten kann ein die relative Schwenkbewegung begrenzendes Zugelement zur Überbrückung des Zwischenraumes zwischen den Schalensegmenten vorgesehen sein. Eine Anpassung des Anpressdruckes über das Zugelement findet nicht statt, das Zugelement verhindert nur eine zu starke Ausschwenkung der Schalensegmente voneinander weg.

Die WO 2013/191933 A2 betrifft eine Orthese mit einem Behandlungsprogramm. Die Orthese, insbesondere Lumbalorthese, weist eine Spanneinrichtung für ein Spannmittel sowie einen motorischen Antrieb auf. Die Spanneinrichtung verlagert zwei Orthesensegmente aufeinander zu oder entspannt die Spanneinrichtung, so dass durch eine aufgebrachte Rückstellkraft die Orthesensegmente voneinander entfernt werden können. Die Orthesensegmente werden im Bauchbereich über einen Klettverschluss miteinander gekoppelt, im Rückenbereich ist die Spannvorrichtung angeordnet, die die Orthese automatisch spannt oder entspannt. Drucksensoren sind in der Orthese angeordnet, um die Spannung zu messen. Auch werden Änderungen der Position des Nutzers detektiert, wird die Spannung der Spanneinrichtung automatisch angepasst. Durch periodisches Spannen und Lösen der Spanneinrichtung kann eine Massagefunktion bereitgestellt werden.

Die US 2014/0068838 A1 betrifft ein motorisiertes Spannsystem für Schuhe, Haltungskorrektureinrichtungen, Rucksäcke, Kopfbedeckungen oder Orthesen. Der Motor stellt über ein Spannmittel, beispielsweise ein Kabel oder ein Seil, die gewünschte Spannung ein. Die Spannung kann über eine Fernbedienung eingestellt werden.

Die US 2014/0121783 A1 betrifft ein einstellbares Prothesenschaftsystem, wobei dieses zumindest einen Flügel und eine an den Flügel gekoppelte Komprimierungsvorrichtung aufweist. Die Komprimierungsvorrichtung presst den Flügel gegen Knochenstrukturen bedeckendes Gewebe. Dieser Anpressdruck vermindert die Bewegung des Flügels relativ zu den darunterliegenden Knochenstrukturen.

Die US 2010/0274364 A1 betrifft einen einstellbaren Prothesenschaft. Der Schaft weist einen Grundkörper mit einem Fenster und einer in dem Fenster angeordneten Platte auf. Der Grundkörper und die Platte bilden einen Hohlraum, in den der Gliedmaßenstumpf einführbar ist. Ein Schnürsystem ist an sowohl den Grundkörper als auch die Platte gekoppelt und bewegt die Platte in Bezug auf den Grundkörper, um das Volumen des Hohlraums anzupassen. Ein Spannmechanismus hält das Schnürsystem, um die Platte zu positionieren und zu fixieren.

Die US 2015/0265434 A1 betrifft eine flexible distale Schale als Bestandteil eines modularen Prothesenschafts. Die flexible Schale ist in einer äußeren Stützstruktur des Prothesenschafts angeordnet und zur Aufnahme eines distalen Bereichs des Gliedmaßenstumpfes eingerichtet.

Die US 2012/02090404 A1 betrifft eine Vorrichtung für die Versorgung eines Gliedmaßenstumpfs nach der Amputation. Die Vorrichtung weist eine erste Schale auf, die an einem distalen Ende ein als halboffener Hohlraum ausgebildetes Endteil aufweist, wobei die Schale eine sich von dem proximalen Ende erstreckende Öffnung aufweist und eingerichtet ist, einen Gliedmaßenstumpf aufzunehmen. Ein zweites Schalenteil wird zumindest teilweise überlappend mit dem ersten Schalenteil in die Öffnung des ersten Schalenteils eingebracht, um die Kontur der Vorrichtung zu schließen. Durch geeignete Befestigungsmittel, etwa die Vorrichtung umschließende Klettelemente, werden die beiden Schalenteile an der Gliedmaße festgelegt.

Die Festlegung des Prothesenschaftes an einem Stumpf, gegebenenfalls an einem Liner, kann schwierig sein, insbesondere für mehrfach versorgte Patienten, die hinsichtlich ihrer manuellen Fertigkeiten eingeschränkt sind.

Aufgabe der vorliegenden Erfindung ist es, einen Prothesenschaft und ein Verfahren bereitzustellen, die ein einfaches Anlegen ermöglichen.

Erfindungsgemäß wird diese Aufgabe durch einen Prothesenschaft mit den Merkmalen des Hauptanspruches sowie ein Verfahren mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

In der Erfindung ist es vorgesehen, dass der Prothesenschaft mit einer proximalen Einführöffnung und einem einen Stumpf zumindest teilweise umgebenden Innenumfang, zumindest einer Anschlussvorrichtung für eine Prothesenkomponente, die an dem Prothesenschaft befestigt ist, zumindest einen Aktuator aufweist, über den der Innenumfang des Prothesenschaftes vergrößerbar ist, wobei der Prothesenschaft über eine elastische Rückstellkraft an dem Stumpf hält und der Aktuator ausgebildet ist, den Innenumfang des Prothesenschaftes entgegen der Rückstellkraft zu vergrößern.. Der Aktuator kann manuell oder motorisch antreibbar oder angetrieben sein, so dass es möglich ist, eine Erweiterung des Innenumfanges des Prothesenschaftes, also das Öffnen des Prothesenschaftes, auch ohne einen Sensor durchzuführen. Die obenstehenden, zu einer Umfangsvergrößerung in Verbindung mit einem Sensor gemachten Ausführungen gelten für einen solchen Prothesenschaft entsprechend, jedoch ohne dass ein Sensor und eine Steuereinrichtung vorgesehen sein müssen. Ein motorischer Antrieb kann durch einen Schalter, eine Fernbedienung oder dergleichen von einem Prothesennutzer aktiviert oder deaktiviert werden, um den Innenumfang des Prothesenschaftes zu vergrößern. Eine manuelle Betätigung oder Betätigbarkeit des Aktuators verringert das Gewicht des Prothesenschaftes und ermöglicht eine einfache Vergrößerung des Innenumfanges durch manuelles Verstellen oder Manipulieren des Aktuators.

Die elastische Rückstellkraft wird durch die Prothesenschaftwand selbst oder durch die Stützelemente ausgebildet. Ebenso kann durch eine elastische Lagerung des Stützelementes oder der Prothesenschaftwand die elastische Rückstellkraft in Richtung auf den Stumpf, also in Richtung auf einen sich verkleinernden Innenumfang aufgebracht werden. Der gewünschte Innendurchmesser wird durch den Grand der Öffnung des Prothesenschaftes durch den Aktuator eingestellt und erzielt. Das Öffnen kann sowohl manuell als auch manuell gesteuert motorisch erfolgen. Ebenso ist es möglich, dass das Öffnen automatisch nach der Detektion entsprechender Sensorwerte wie Lage, Position im Schaft, Position des Schaftes, Druck, Zeitdauer eines Druckes, Druckveränderungen, Sauerstoffsättigung, Durchblutung, Temperatur oder dergleichen ausgeübt wird. Nach dem Öffnen, insbesondere nach einem vollständigen Öffnen, kann der Prothesenschaft über den Stumpf gezogen oder der Stumpf in den Prothesenschaft eingesetzt werden. Durch Nachlassen der zum Öffnen aufgebrachten Kraft, beispielsweise einer entsprechend umgelenkten Zugkraft, durch ein Spreizelement, das an einem Spalt entlang gefahren wird oder durch einen Ring, der entlang eines konisch geformten Schaftes verfahren wird, wird die auf den Stumpf aufgebrachte Anlagekraft eingestellt und der Schaft an den Stumpf angepasst. Eine aktive Betätigung erfolgt somit nur in Richtung des Öffnens des Schaftes, die Rückstellung in Richtung auf den Stumpf und damit eine Verringerung des Innenumfanges erfolgt passiv durch eine elastische Ausgestaltung oder Lagerung der Stützelemente oder der Prothesenschaftwand.

Der Prothesenschaft ist insbesondere mehrteilig, zumindest mit zwei Stützelementen ausgebildet, die den Stumpf zwischen sich aufnehmen, wobei die Stützelemente den Stumpf entweder vollständig umgeben oder zumindest teilweise so umfassen, dass dieser fest in dem Prothesenschaft aufgenommen ist. Die Stützelemente können einander überlappend ausgebildet sein.

Zumindest eine Spanneinrichtung kann an dem Prothesenschaft angeordnet sein, um dessen Innenumfang zu verändern. Die Spanneinrichtung kann als Zugmittel, pneumatisch oder hydraulisch angetriebene Spreiz- oder Spannmittel, als längsverschiebliches Spreiz- oder Schließelement, als verfahrbarer Ring, als Kippelement oder als vertretbares Betätigungselement ausgebildet sein. Das jeweilige Spannmittel wird durch den Aktuator, gegebenenfalls unter Zwischenschaltung von Getriebe, Kraftübertragungseinrichtung oder dergleichen aktiviert oder deaktiviert.

Der Aktuator, z.B. als manueller oder motorischer Antrieb einer Spanneinrichtung, erleichtert das Anziehen des Prothesenschaftes ungemein. Es müssen keine aufwändigen Verschnürungen, Betätigungen von Spanneinrichtungen, Verriegelungen von mechanischen Befestigungseinrichtungen oder dergleichen von Hand vorgenommen werden. Vielmehr wird bei einem motorischen Aktuator der Innenumfang, z.B. über die Spanneinrichtung oder das veränderbare Volumen oder die Spreizeinrichtung auf die gewünschte Spannung eingestellt, ohne dass manuelle Kraft aufgewendet werden müsste.

Die zumindest eine Spanneinrichtung kann als Gurt, Seil, Band, Kabel oder Litze ausgebildet sein oder ein solches Spannelement aufweisen. Grundsätzlich sind alle zugkraftübertragenden Spannelemente sinnvoll einsetzbar, auch druckstarre Spanneinrichtungen oder Spannelemente wie Stäbe, Spangen oder Ringe. Flexible Spanneinrichtungen haben den Vorteil, dass sie sich an verändernde Umfänge leichter anpassen lassen und insgesamt einfacher handhabbar sind. Neben flexiblen Spanneinrichtungen, die vorteilhafterweise unelastisch sind, können auch Zahnriemen, Lochriemen oder Ketten als Spanneinrichtungen oder Spannelemente eingesetzt werden.

Das zumindest eine Zugkraft übertragende Spannelement kann in Ösen, Kanälen und/oder um zumindest eine Umlenkrolle geführt sein, um beispielsweise ähnlich einem Flaschenzug eine Kraft-Weg-Übersetzung zu erreichen. Durch das Umlenken flexibler Spannelemente kann der Angriffspunkt der Spannkraft definiert werden. Durch eine Vielzahl von Umlenkrollen kann die Spannkraft vielfach verteilt und somit eine gleichmäßige Druckverteilung erreicht werden. Darüber hinaus ist es durch Umlenkrollen möglich, mit nur einer Spanneinrichtung Spannkräfte an mehreren Stellen aufzubringen, wodurch sich die Teilezahl verringert und eine erleichterte Montage verwirklichen lässt. Durch die Führung innerhalb von Kanälen, die sich innerhalb des Schaftes, beispielsweise in den Stützelementen befinden können, wird das Risiko minimiert, dass Kleidungsstücke oder andere Gegenstände zwischen der Spanneinrichtung und dem Stützelement eingeklemmt werden. Ebenso ist es möglich, durch eine Umlenkung des Spannelementes ein Aufspreizen des Prothesenschaftes zu bewirken, so dass durch Aufrollen oder Aufwickeln eine Innenumfangsvergrößerung bewirkt wird. Dazu wird das Spannelement um eine Umlenkstelle geführt, die jenseits der Befestigungsstelle liegt, an dem das freie, nicht aufgewickelte Ende befestigt ist. Dadurch wird bei einem Aufwickeln beispielsweise ein Stützelement von einem anderen weggezogen.

Es können mehrere Aktuatoren oder Spanneinrichtungen unabhängig voneinander entlang der proximal-distal-Erstreckung des Prothesenschaftes angeordnet sein, um unabhängig voneinander verschiedene Anpressdrücke auf den Stumpf durch eine Veränderung des Schaftinnenumfanges einstellen zu können.

Die zumindest eine Spanneinrichtung wird vorteilhafterweise in Umfangsrichtung des Stumpfes wirkend angeordnet, um so einen radial wirkenden Anpressdruck des Stützelementes oder der Stützelemente auf den Stumpf zu bewirken. Bei einer einteiligen Ausgestaltung des Prothesenschafes mit der Schaftwand als Stützelement wirkt die Spanneinrichtung aufwickelnd oder die Überdeckung vergrößernd, so dass sich der Innenumfang verringert, alternativ wird bei einer gegenläufigen Bewegung der Innenumfang vergrößert, zumindest bereichsweise, so dass der Schaft gelockert oder geöffnet wird.

Die Stützelemente oder die Schaftwand selbst können elastisch ausgebildet sein, um beispielsweise bei einer Entspannung der Spanneinrichtung eine Rückstellkraft bereitzustellen, durch die es ermöglicht wird, dass sich der Prothesenschaft selbsttätig öffnet und somit der Patient leicht aus dem Prothesenschaft heraussteigen kann. Ebenso kann umgekehrt eine auf den Stumpf wirkende Vorspannung durch die Schaftwand oder die Stützelemente aufgebracht sein, die durch eine Aufspreizeinrichtung aufgehoben wird, um aus dem Schaft auszusteigen oder um den auf den Stumpf wirkenden Druck zu reduzieren. Die Stützelemente können auch elastisch an dem Endstück gelagert sein, beispielsweise gelenkig entgegen einer Federkraft. Ebenso ist es möglich, dass die Stützelemente einstückig mit dem Endstück ausgebildet sind und zum Schließen des Prothesenschaftes oder zum Anlegen des Prothesenschaftes aus einer entfernt voneinander liegenden Ausgangsposition aufeinander zu bewegt werden.

Um einen möglichst gleichmäßig auf den Stumpf wirkenden Anpressdruck der Schaftwand oder der Stützelemente zu erreichen, können diese in Umfangsrichtung einander überlappend ausgebildet sein, so dass sich zumindest im angelegten, gespannten Zustand der Prothesenschaft sich vollständig um den Stumpf herum anlegt und einen quasi geschlossenen Querschnitt ausbildet. Dadurch wird verhindert, dass Teile des Stumpfes durch einen Freiraum zwischen den Stützelementen oder in der Schaftwand herausgedrückt werden, wenn der Innenumfang verringert wird.

Die Schaftwand oder die Stützelemente können in Richtung voneinander weg mit einer Federkraft belastet oder im ungespannten Zustand sich in Proximalrichtung konisch erweiternd angeordnet sein, wodurch das Einsteigen in dem Schaft bzw. Anlegen des Prothesenschaftes an den Stumpf erleichtert wird. Im nicht gespannten Zustand ergibt sich somit eine trichterförmig oder konisch erweiterte Form, die das Einführen eines Stumpfes erleichtert. Erst nach Einführen des Stumpfes in den Prothesenschaft wird die Spanneinrichtung über den Aktuator gespannt oder die Spreizeinrichtung zurückgefahren, wodurch der Innenumfang verringert und z.B. die Stützelemente in Richtung aufeinander zu bewegt, entweder gegen eine Federkraft oder gegen eine elastische Rückstellkraft aufgrund der Materialverformung, so der Prothesenschaft fest an dem Stumpf anliegt.

In dem Prothesenschaft kann ein distaler Kontaktschalter oder Sensor zur Detektion eines eingeführten Stumpfes angeordnet sein. Der distale Kontaktschalter oder distale Sensor detektiert, ob der Stumpf vollständig oder zumindest bis zum einem vorgegebenen Punkt in den Prothesenschaft eingeführt ist. Sofern der Kontaktschalter oder Sensor die entsprechende Position des Stumpfes detektiert, kann automatisch der Aktuator aktiviert oder deaktiviert werden, um den Innenumfang zu verringern, wodurch sich ein selbstschließender oder selbstanlegender Prothesenschaft realisieren lässt. Der Prothesenschaft passt sich dann automatisch an die Außenkontur des Stumpfes an. Der distale Kontaktschalter oder Sensor ist vorteilhafterweise an dem Endstück angeordnet, das auch den distalen Abschluss des Einführraumes des Stumpfes bildet, kann aber auch oberhalb davon angeordnet sein.

Eine Weiterbildung der Erfindung sieht vor, dass zumindest ein Motorstromsensor oder ein innenliegender Drucksensor zur Erfassung des durch ein Stützelement auf den Stumpf aufgebrachten Druckes an der Schaftwand oder dem Stützelement angeordnet ist. Innenliegende Drucksensoren ermitteln den Anpressdruck des Stützelementes an dem Stumpf. Dazu ist es sinnvoll, den Drucksensor auf der Innenseite, also der dem Stumpf zugewandten Seite der Schaftwand oder des Stützelementes anzuordnen. Alternativ zu einer direkten Druckmessung ist es möglich und vorgesehen, über einen Motorstromsensor den Motorstrom zu messen, der proportional zu dem Motormoment ist und damit auch proportional zu der Kraft, mit welcher die Spanneinrichtung gespannt oder der Innenumfang verringert wird. Dadurch lässt sich auf den Anpressdruck des Stützelementes an den Stumpf oder den Schaftinnendruck schließen. Über den oder die Sensoren ist es möglich, eine optimierte Druckverteilung und somit einen optimierten Sitz des Prothesenschaftes an dem Stumpf zu erreichen. Insbesondere in Verbindung mit einem distalen Kontaktschalter, der die motorischen Antriebe aktiviert, ist es möglich, nach dem einfachen Einführen des Stumpfes in den Prothesenschaft ein automatisches Schließen oder Öffnen durchzuführen. Die Drucksensoren oder Motorstromsensoren erfassen den Druck am Stumpf. Überschreitet der Druck einen eingestellten Wert oder erreicht diesen, wird über eine Steuereinrichtung das weitere Spannen der Spanneinrichtung oder das Reduzieren einer Spreizung gestoppt. Somit wird stets der vorgesehene Anpressdruck bei einem Einsteigen oder Anlegen des Prothesenschaftes erreicht. Verändert sich der Schaftinnendruck, beispielsweise durch die Volumenschwankungen aufgrund längerer Belastungen oder Lagerveränderungen, wird dies über die Drucksensoren oder den Motorstromsensor erfasst. Es besteht dann die Möglichkeit, entweder die Spanneinrichtung oder die Spreizeinrichtung oder die anderen, den Innenumfang verändernden Einrichtungen entsprechend zu lösen oder anzuziehen, um eine Volumenvergrößerung oder Volumenverringerung im Stumpf auszugleichen.

Mehrere Drucksensoren können proximal-distal verteilt an der Schaftwand oder zumindest einem Stützelement angeordnet sein. Bei mehreren Motoren können mehrere Motorstromsensoren an dem Prothesenschaft angeordnet sein, so dass unabhängig voneinander über die proximal-distal-Erstreckung des Prothesenschaftes ein angepasster Druck einstellbar ist. Es können auch Druck- und Motorstromsensoren in Kombination eingesetzt werden. Es können weiterhin Sauerstoffsättigungssensoren, Durchblutungssensoren und/oder Temperatursensoren eingesetzt werden.

Grundsätzlich ist es möglich, die Spanneinrichtung oder auch mehrere Spanneinrichtungen über einen Motor zu spannen. Eine unabhängig voneinander einstellbare Spannung der Spanneinrichtungen erfolgt vorteilhafterweise über mehrere Motoren, die an dem Prothesenschaft angeordnet sind. Mit einem Motor können unabhängig voneinander agierende Spanneinrichtungen zwar verstellt werden, jedoch ist die ausgeübte Spannkraft der Spanneinrichtungen nicht unabhängig voneinander, zwar können über Getriebe unterschiedliche Verstellwege der Spanneinrichtungen realisiert werden, bei nur einem Motor sind diese Verstellwege jedoch miteinander gekoppelt. Durch mehrere Motoren ist eine individuelle Einstellung der mehreren Spanneinrichtungen möglich, wodurch sich verschiedene Druckverteilungskennlinien, Druckmuster oder auch alternierende Druckkräfte während des Tragens realisieren lassen.

An dem Prothesenschaft kann zumindest eine Steuereinrichtung zur Aktivierung und Deaktivierung des zumindest einen Aktuators, insbesondere Motors angeordnet sein. Die Steuereinrichtung kann direkt mit einem Schalter, per Gestensteuerung oder per Fernbedienung ansteuerbar sein, um beispielsweise auf Knopfdruck die Öffnungsfunktion oder Schließfunktion auszuführen. Die Steuereinrichtung kann auch über andere Signale aktiviert werden, beispielsweise akustische Signale, Bewegungsmuster oder eine andere Art und Weise der Fernbedienung.

An dem Prothesenschaft oder einer angeschlossenen Prothesenkomponente kann zumindest ein Lagewinkelsensor, Winkelsensor, Beschleunigungssensor, Schalter, Sauerstoffsättigungssensor, Durchblutungssensor, Temperatursensor und/oder Inertialsensor angeordnet sein, der oder die mit der Steuerungseinrichtung gekoppelt ist/sind. Über die Sensoren können die gegenwärtigen Einsatzbedingungen der Prothese erfasst und eine Anpassung des Druckes in Abhängigkeit von den Einsatzbedingungen vorgenommen werden. Wird beispielsweise eine besonders hohe Aktivität detektiert, kann der Anpressdruck verstärkt und die Spanneinrichtungen gespannt werden, um einen optimalen Halt zu gewährleisten. In Aktivitätspausen kann der Anpressdruck reduziert werden. Ebenso ist es möglich, für gefährdete Nutzer automatisch den Anpressdruck nach einer gewissen Zeit zu reduzieren, beispielsweise für Diabetiker, um zu vermeiden, dass zu hohe Drücke über einen zu hohen Zeitraum auf den Stumpf ausgeübt werden. Dadurch kann eine bestimmte Tragezeitbegrenzung verwirklicht werden. Die Reduzierung des Druckes kann angezeigt oder ausgegeben werden, beispielsweise optisch, akustisch oder haptisch. Es ist Rückmeldesystem für den Patienten in den Schaft oder in einer gesonderten Komponente wie Tablet, Mobiltelefon, Anzeigeelement oder Warneinrichtung vorhanden. Das Rückmeldesystem kann als App, Lampe, Vibrationserzeuger oder ähnliches dem Patienten eine Rückmeldung geben. Die Rückmeldung über einen zu hohen Druck auf den Stumpf oder über einen zu langen Zeitraum ist insbesondere für Diabetiker wichtig, die zum Teil ein vermindertes Schmerzempfinden und ein hohes Risiko für Gefäßverengungen haben. Auch beim Sitzen kann der Anpressdruck automatisch reduziert werden, wird die Prothese wieder genutzt, beispielsweise bei Prothesen der unteren Extremitäten beim Aufstehen und Gehen, werden die Drücke automatisch erhöht. Zur Erfassung der Stumpfposition im Schaft können Drucksensoren oder Berührungssensoren verwendet werden, letzte ähnlich einem Touch-Screen.

Dem Aktuator oder der Spanneinrichtung kann eine Ratsche oder ein Spannhebel zugeordnet sein, um eine Sperrung in der eingestellten Position mechanisch zu bewirken.

Weiterhin ist es möglich, eine mechanische Entsperreinrichtung an dem Prothesenschaft anzuordnen, um beispielsweise im Falle des Ausfalls der Energieversorgung für die Aktuatoren oder Motoren ein Ablegen des Prothesenschaftes zu ermöglichen. Im geschlossenen Zustand kann der auf den Druck aufgebrachte Anpressdruck in proximaler Richtung abnehmen, um ungewünschte Blutstauungen im distalen Bereich des Stumpfes zu vermeiden.

In einer Weiterbildung der Erfindung ist es vorgesehen, dass der Prothesenschaft in einem 3D-Druckverfahren hergestellt wird. Ein Endstück, soweit vorhanden, die Stützelemente und/oder die Schaftwand sind im 3D-Druckverfahren hergestellt. Ebenso können weitere Komponente des Prothesenschaftes in einem 3D-Druckverfahren hergestellt werden, beispielsweise die Spannelemente wie Zugfäden, Gurte oder Hebel, die Ösen oder Tunnel zur Führung von Spannelementen, Führungen, Spreizelemente, Aktuatoren oder Aufnahmen für Aktuatoren. Dadurch lassen sich eine Vielzahl von Komponenten in den Prothesenschaft integrieren.

Das Verfahren zur Steuerung einer Anpassung des Innenumfanges eines Prothesenschaftes, wie er vorstehend beschreiben wurde, sieht vor, dass bei einer Detektion einer vorbestimmten Position des Stumpfes, zum Beispiel einer bestimmten Raumlage oder einer bestimmten Position innerhalb des Prothesenschaftes und/oder bei Detektion einer auf den Stumpf ausgeübten Druckkraft oder den zumindest einen Sensor automatisch der Aktuator aktiviert oder deaktiviert wird. Die Spanneinrichtung wird beispielsweise automatisch motorisch gespannt oder entspannt. Hierdurch wird das Anlegen und Ablegen des Prothesenschaftes automatisiert und erleichtert.

Eine Weiterbildung der Erfindung sieht vor, dass die Spanneinrichtung nur bis zu einem festgelegten Anpressdruck gespannt wird, der Anpressdruck wird sensorisch überwacht, beispielsweise durch direkte Drucksensoren oder Motorstromsensoren.

Der Anpressdruck der Stützelemente an dem Stumpf kann erfasst werden, wobei der Anpressdruck in Abhängigkeit von der Aktivität, einer Zeitgröße, einer Position des Prothesenschaftes oder einer Anpressdruckänderung verändert wird. Treten hohe Beschleunigungskräfte auf den Prothesenschaft auf, beispielsweise durch eine verstärkte Aktivität beim Sport, kann der Anpressdruck erhöht werden. Wird ein bestimmter Anpressdruck über eine bestimmte Zeitspanne hinweg aufgebracht, kann über ein Zeitsignal eine Anpressdruckreduzierung eingeleitet werden, um Schädigungen des Stumpfgewebes zu vermeiden. Befindet sich der Prothesenschaft in einer bestimmten Stellung, bei Prothesenschäften unterer Extremitäten beispielsweise in einer waagerechten Stellung, und wird diese über einen bestimmten Zeitraum beibehalten, kann auf eine bestimmte Aktivität geschlossen werden, beispielsweise Sitzen oder Liegen, so dass eine Verringerung des Anpressdruckes vorgenommen werden kann. Verändert sich der Anpressdruck über einen Schwellwert hinaus, beispielsweise durch eine Volumenvergrößerung oder Volumenverringerung, kann ebenfalls eine Anpassung des Anpressdruckes veranlasst werden, um eine Annäherung an einen einmal festgelegten, möglichst optimierten Anpressdruck zu erreichen.

Zum Öffnen des Prothesenschaftes können Stützelemente durch den Aktuator voneinander weg verlagert werden, so dass durch den Aktuator der Prothesenschaft aktiv geöffnet wird, um den Innenumfang zu vergrößern. Gleiches kann bei einer Ausgestaltung mit einer Prothesenschaftwand geschehen, indem durch den Aktuator die Schaftwand auseinandergezogen, auseinanderbewegt, auseinandergedrückt oder aufgespreizt wird, so dass ein Spalt vergrößert oder eine Überdeckung verringert wird. Alternativ oder ergänzend kann zur Umfangsveränderung eine gegen eine elastische Vorspannkraft der Stützelemente oder der Schaftwand wirkende Spanneinrichtung entspannt werden. Der Prothesenschaft wird dabei durch das elastische Verhalten der Stützelemente, der Schaftwand oder deren Lagerung aufgeweitet, die in Richtung auf einen vergrößerten Innenumfang hin mit einer Vorspannkraft beaufschlagt sein können.

In Ergänzung oder als Alternative werden zum Schließen des Prothesenschaftes oder zur Verringerung des Innenumfanges Stützelemente oder eine Schaftwand des Prothesenschaftes durch den Aktuator oder durch ihr elastisches Verhalten aus einer aufgeweiteten Stellung in eine gespannte Stellung mit einem gegenüber der Ausgangsstellung verringerten Innenumfang aufeinander verlagert. Das Schließen erfolgt aktiv durch einen Aktuator, der eine Schließeinrichtung betätigt und die Stützelemente aufeinander zu verlagert oder eine Schaftwand zusammenbewegt. Dies kann durch Spannelemente, das Verschieben zumindest eines Schiebers, einer Spange oder eines Ringes erfolgen. Alternativ oder ergänzend sind die Stützelemente, die Schaftwand oder deren Lagerung dergestalt elastisch ausgebildet, dass bei einem Wegfall einer Spreizkraft oder einer Sperre der Innenumfang durch das elastische Verhalten verringert wird.

In einer Variante der Erfindung ist es vorgesehen, dass zur Anpassung des Innenumfanges eines Prothesenschaftes der Prothesenschaft geöffnet wird, beispielsweise indem Stützelemente durch einen Aktuator voneinander weg verlagert werden, eine Schaftwand auseinandergezogen oder zur Umfangsveränderung eine gegen eine elastische Vorspannkraft der Stützelemente oder der Schaftwand wirkende Spanneinrichtung entspannt wird. Das Öffnen eines Prothesenschaftes mit einer veränderlichen Innenumfang erfolgt somit entweder manuell oder motorisch durch aktives voneinander weg Verlagern von Komponenten eines Prothesenschaftes, beispielsweise verlagerbare Stützelemente, oder durch Auseinanderziehen einer Schaftwand, beispielsweise durch Aufweiten eines Spaltes oder durch eine Verringerung eines Überdeckungswinkels einer sich überlappenden Schaftwand. Ebenfalls ist es möglich, dass zur Umfangsveränderung eine Spanneinrichtung entspannt wird, wobei die Spanneinrichtung gegen eine elastische Vorspannkraft der Stützelemente der Schaftwand wirkt. Das Öffnen des Prothesenschaftes kann somit auch ohne Sensor erfolgen.

Bevorzugt wirkt der Aktuator über Spreizelemente, Zugmittel, Spanneinrichtung oder dergleichen aktiv gegen eine Vorspannkraft, die in Richtung auf eine Umfangsverringerung des Prothesenschaftes wirkt. Der Schaft hält durch die elastische Rückstellkraft der Stützelemente oder der Schaftwand an dem Stumpf. Die erforderliche Anpresskraft oder Haltekraft wird durch die Rückstellkraft aufgebracht. Die Einstellung des Anpressdruckes oder des optimalen Innenumfanges wird durch ein angepasstes Öffnen beziehungsweise Nachgeben des Aktuators gegen die nach innen wirkende Vorspannkraft bewirkt. Bei einem ausreichenden, insbesondere vollständigen Öffnen des Prothesenschaftes kann der Stumpf in den Innenumfang eingesetzt werden, und durch Nachlassen einer Zugkraft, durch Verfahren eines Spreizelementes oder Aufheben einer Sperre wird durch die Rückstellkraft der Schaft an den Stumpf angepasst und angepresst. Geöffnet wird der Prothesenschaft aktiv, die Rückstellung erfolgt passiv durch die Vorspannkraft.

Der Prothesenschaft in einer Variante mit einer proximalen Einführöffnung und einem einen Stumpf zumindest teilweise umgebenen Innenumfang, zumindest einer Anschlussvorrichtung für eine Prothesenkomponente, die an dem Prothesenschaft befestigbar und zumindest einem Aktuator, über den der Innenumfang des Prothesenschaftes veränderbar ist, sieht zumindest einen Sensor vor, der mit einer Steuerungseinrichtung gekoppelt ist, wobei die Steuerungseinrichtung mit dem Aktuator verbunden ist und diesen in Abhängigkeit von den empfangenen Sensorsignalen aktiviert oder deaktiviert.

Durch die Kopplung eines Aktuators zur Veränderung des Innenumfanges eines Prothesenschaftes mit einem Sensor über eine Steuerungseinrichtung dergestalt, dass der Aktuator in Abhängigkeit von empfangenen Sensorsignalen aktiviert oder deaktiviert wird, ermöglicht ein automatisches Spannen oder Entspannen des Prothesenschaftes beziehungsweise der Anlagekraft des Prothesenschaftes an den Stumpf, so dass beispielsweise der Prothesenschaft geöffnet oder geschlossen werden kann, um ein Aussteigen und Einsteigen zu erleichtern und ein automatischen Anlegen zu ermöglichen. Darüber hinaus ist es möglich, automatisch oder auch willentlich Volumenschwankungen innerhalb des Stumpfes auszugleichen, indem beispielsweise bei einem sich vergrößernden Stumpfvolumen während der Tragedauer der Innenumfang vergrößert wird, um den Anpressdruck zu verringern. Ebenso ist es möglich, den Innenumfang bei einer Detektion von besonderen Belastungen, beispielsweise beim Sport, zu verringern, um eine festere Anlage des Prothesenschaftes an den Stumpf bereitzustellen. Darüber hinaus können periodische Veränderungen des Innenumfanges vorgenommen werden, um einen Massageeffekt zu erzielen. Auch kann bei der Detektion von Ruhephasen, beispielsweise während des Sitzens, automatisch der Innenumfang vergrößert werden. Das Öffnen und Schließen des Prothesenschaftes kann somit selbstständig erfolgen.

Der Aktuator kann als Antrieb für eine Pumpe, ein Verschiebelement, einen Hebel, eine Wickeleinrichtung für Zugmittel, ein Spreizelement, ein Getriebe, als Antrieb zur Aktivierung einer Formgedächtnislegierung oder eine elektroaktiven Polymers oder als schaltbarer Magnet ausgebildet sein. Insbesondere kann der Aktuator als Motor ausgebildet sein und beispielsweise eine Spanneinrichtung antreiben, die beispielsweise ein Zugmittel wie Gurt, Seil, Band, Kabel oder Litze aufweist und dessen effektive Länge vergrößert oder verkleinert und dadurch den Prothesenschaft in seinem Innenumfang verändert. Grundsätzlich ist es auch vorgesehen, das der Aktuator manuell betrieben oder manuell angesteuert werden kann, beispielsweise durch einen Schalter, so dass keine Steuereinrichtung und kein Sensor vorhanden sein müssen. Der zumindest eine Aktuator kann auch eine Pumpe antreiben, über die ein veränderbares Volumen, das an oder in dem Prothesenschaft angeordnet oder ausgebildet ist, verändert werden. So kann beispielsweise bei einem geschlossenen Prothesenschaft ein auf der Innenseite angeordnetes Volumen vorhanden sein, das mit Druckluft oder einem Hydraulikfluid, das an einem außenseitigen Reservoir angeordnet ist, gefüllt werden kann, um so den Innenumfang zu verändern. Dazu benötigt der Prothesenschaft ein Widerlager, das beispielsweise durch den geschlossenen Querschnitt oder aber durch eine rigide Spanneinrichtung oder Begrenzungseinrichtung, beispielsweise ein festeingestelltes Zugmittel bereitgestellt wird. Ebenso ist es möglich, dass über eine Volumenveränderung bei einem mehrteiligen Prothesenschaft mit mehreren Stützelementen, die relativ zueinander verlagerbar sind, eine Innenumfangsveränderung realisiert wird. Dies kann entweder durch Aufbringen einer Verlagerungskraft auf die Stützelemente durch das sich verändernde Volumen erfolgen, beispielsweise durch Aufblasen, oder durch ein Befüllen eines gegen ein Widerlager drückendes Volumen oder aber durch innenseitige Volumenvergrößerung. Ebenfalls ist es möglich, das Volumen mechanisch zu vergrößern, beispielsweise durch Aufstellen von gelenkig gelagerten Hebeln oder Fasern, die sich bei Verlagerung beispielsweise eines Schiebers aufstellen, gegen eine Volumenwand drücken und dadurch eine Volumenveränderung des Volumens bewirken. Ebenso ist es möglich, dass ein um den Prothesenschaft herum gelegter Ring längsverschieblich gelagert ist. Durch eine konische Ausgestaltung des Prothesenschaftes oder eine konische Gestaltung einer Ringführung an der Außenseite ist es möglich, durch eine Längsverschiebung Stützelemente, die Teil eines mehrteiligen Prothesenschaftes sind, aufeinander zu zu verlagern oder voneinander weg zu bewegen. Dadurch kann eine nach innen gerichtete Verschwenkung oder außen gerichtete Aufspreizbewegung verwirklicht werden. Neben einer mehrteiligen Ausgestaltung des Prothesenschaftes ist es auch möglich, diesen einteilig auszubilden und mit einem Schlitz zu versehen, gegebenenfalls mit überlappenden Seitenkanten, um ein vollständiges Umfassen des Prothesenstumpfes zu ermöglichen und gleichzeitig eine Umfangsveränderung zu ermöglichen. Der Prothesenschaft ist dann spiralförmig ausgebildet, eine Veränderung des Innenumfanges wird dadurch erreicht, dass der Wicklungswinkel oder Überdeckungswinkel der beiden Seitenkanten verändert wird.

Ebenso ist es möglich, dass der Aktuator als schaltbarer Magnet ausgebildet ist, über den eine entsprechende Verstellung mechanischer, magnetorheologischer oder sonstiger Komponenten erfolgen kann.

Nachfolgend werden Ausführungsbeispiele der Erfindung anhand der beigefügten Figuren näher erläutert. Es zeigen:
- Figur 1: eine Draufsicht auf einen Prothesenschaft und Druckverteilungskennlinie;
- Figur 2: einen geöffneten Prothesenschaft mit einer Spanneinrichtung und einem Motor;
- Figur 3: einen geschlossenen Prothesenschaft gemäß Figur 2;
- Figur 4: einen geöffneten Prothesenschaft mit mehreren Spanneinrichtungen und Motoren;

- Figur 5: einen geschlossenen Prothesenschaft gemäß Figur 4 ;
- Figur 6: ein Schaltbild;
- Figur 7: eine schematische Darstellung einer Einrichtung zum Öffnen eines Schaftes;
- Figur 8: einen Prothesenschaft in Detailansicht in geschlossener Stellung; sowie
- Figur 9: das Funktionsprinzip gemäß Figur 8 in geöffneter Stellung.

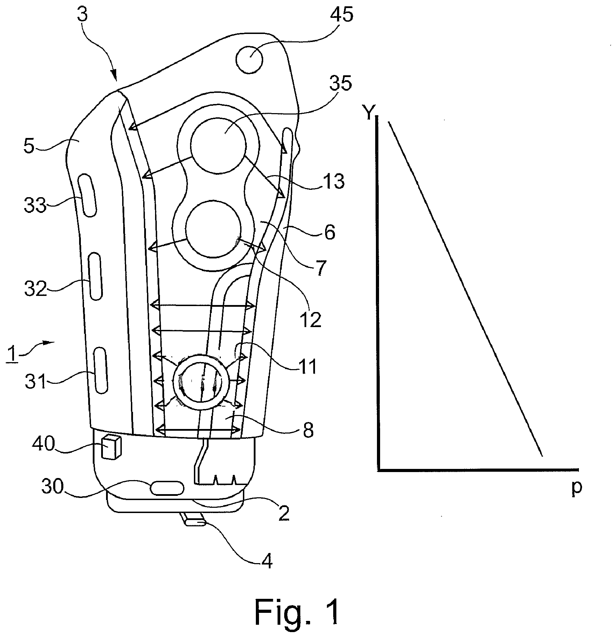

In der Figur 1 ist in einer Draufsicht ein Prothesenschaft 1 dargestellt, der im dargestellten Ausführungsbeispiel als Oberschenkelschaft zur Aufnahme eines Oberschenkelstumpfes ausgebildet ist. Grundsätzlich ist es auch möglich, den Prothesenschaft 1 als Unterschenkelschaft oder als ein Prothesenschaft für eine obere Extremität auszugestalten. Der Prothesenschaft 1 weist an seinem distalen Ende ein Endstück 2 auf, das den distalen Abschluss eines Aufnahmeraumes für einen Stumpf bildet. Das distale Endstück 2 liegt einer proximalen Öffnung 3 gegenüber. Der Prothesenschaft 1 ist im Wesentlichen trichterförmig ausgebildet, umschließt den nicht dargestellten Stumpf im Wesentlichen vollumfänglich und bildet einen unteren Abschluss, so dass ein Stumpf, gegebenenfalls zusammen mit einem über dem Stumpf gezogenen Liner nur bis zu dem distalen Endstück 2 einführbar ist.

An dem distalen Endstück ist ein Anschlussmittel 4 für weitere Prothesenkomponenten angeordnet oder befestigt. Die Anschlussmittel 4 sind beispielsweise Pyramidenadapter, formschlüssige Aufnahmen, Schraubaufnahmen, Bolzen oder andere Einrichtungen, mit denen weitere Prothesenkomponenten an dem Prothesenschaft 1 befestigt werden können. Im Ausführungsbeispiel des Prothesenschaftes 1 als Oberschenkelschaft wird an das distale Endstück 2 ein Prothesenkniegelenk über das Anschlussmittel 4 festgelegt.

Bei der Ausgestaltung des Prothesenschaftes als Unterschenkelschaft ist als weitere Prothesenkomponente ein Prothesenfuß oder ein Unterschenkelrohr an dem Anschlussmittel 4 festgelegt, bei der Ausgestaltung als Unterarmschaft eine Prothesenhand.

Das Endstück 2 kann als ebene Platte oder als tassenförmige Aufnahme und Abschluss des Prothesenschaftes 1 ausgebildet sein. Neben einer bevorzugt formstabilen Ausgestaltung des Endstückes 2, beispielsweise aus Metall, Kunststoff oder dergleichen, kann das Endstück 2 auch flexibel ausgestaltet sein. An das Endstück 2 schließen sich in Proximalrichtung Stützelemente 5, 6, 7, 8 an, die entweder einstückig mit dem Endstück 2 ausgebildet oder separat angefertigt und daran befestigt sind. Die Stützelemente 5, 6, 7, 8 sind so angeordnet, dass sie einen Aufnahmeraum zwischen sich bilden, in dem der nicht dargestellte Stumpf durch die Einführöffnung 3 eingeführt werden kann. Die Stützelemente 5, 6, 7, 8 umgeben den Stumpf vollständig. Aufgrund der segmentweisen Ausgestaltung der Stützelemente 5, 6, 7, 8 und einer entweder beweglichen Lagerung an dem Endstück 2 oder an einer innewohnenden Elastizität, beispielsweise aufgrund der Materialwahl oder einer Materialschwächung im Bereich des Endstückes 2, ist es möglich, die Stützelemente 5, 6, 7, 8 voneinander weg und aufeinander zu zu verlagern, um den Prothesenschaft aufzuweiten bzw. die Einführöffnung 3 und den Aufnahmeraum zu verkleinern. Der Innenumfang des Prothesenschaftes 1 wird durch die Verlagerung der Stützelemente infolge der Aktivierung oder Deaktivierung eines Aktuators verändert.

An der Vorderseite des Prothesenschaftes 1 gemäß dem dargestellten Ausführungsbeispiel sind drei Spanneinrichtungen 11, 12, 13 angeordnet, über die zumindest das laterale Stützelement 6 in Richtung auf das mediale Stützelement 5 verlagert werden kann. Das Stützelement 7 ist im Wesentlichen frontal orientiert, ein weiteres Stützelement 8 ist mit einem Scharnier an dem Endstück 2 festgelegt und dient zur verbesserten Frontalabstützung. Es ist möglich, dass das laterale Stützelement 6 an dem weiteren Stützelement 8 befestigt ist.

Die Spanneinrichtungen oder Spannelemente 11, 12, 13 sind im dargestellten Ausführungsbeispiel als Schnüre, Kabel oder Seile ausgebildet, ebenso ist es möglich, dass statt der dargestellten Spanneinrichtungen 11, 12, 13 in Seilform diese auch in Gurtform oder auch Stabform, Kettenform, Lochbandform oder einer anderen Zugmittelform ausgebildet ist. Wesentlich ist, dass die Spanneinrichtung dazu geeignet ist, die Stützeinrichtungen aufeinander zu zu verlagern, also Zugkräfte aufzubringen, wobei aufgrund der flexiblen und vorzugsweise unelastischen Ausgestaltung der Spanneinrichtungen 11, 12, 13 überwiegend zirkuläre Kräfte wirken. Durch die Verlagerung der Stützelemente 5, 6, 7, 8 aufeinander zu wird der Innenumfang des Prothesenschaftes verringert.

Die Spanneinrichtungen 11, 12, 13 sind in Ösen an den einander gegenüberliegenden Rändern der Stützelemente 5, 6 geführt und nach Art einer Schuhschnürung angeordnet. Insgesamt sind drei voneinander unabhängige Spanneinrichtungen 11, 12, 13 an dem Prothesenschaft 1 angeordnet, die in proximal-distal-Richtung versetzt zueinander angeordnet sind, wodurch es möglich wird, dass in unterschiedlichen Höhen unterschiedliche Drücke auf den Stumpf ausgeübt werden.

Die Spanneinrichtungen 11, 12, 13 sind über einen Aktuator, im Ausführungsbeispiel einen Motor spannbar, dazu sind die Spanneinrichtungen auf einer Rolle aufgewickelt, die mit dem Motor gekoppelt ist, beispielsweise über ein Getriebe, so dass relativ kleine Motoren mit hohen Drehzahlen verwendet werden können, was Bauraum und Gewicht spart. Werden die nicht dargestellten Motoren aktiviert, wird die jeweilige Spanneinrichtung 11, 12, 13 aufgewickelt, die Stützeinrichtungen 5, 6, 7, 8 werden aufeinander zu bewegt und der Prothesenschaft 1 schließt sich um den Stumpf.

In dem Prothesenschaft 1 ist weiterhin eine Steuereinrichtung 40 angeordnet, über die die Aktuatoren oder Motoren bzw. der Aktuator oder der Motor, wenn nur ein Aktuator oder Motor verwendet wird, angesteuert werden. Die Steuereinrichtung 40 ist mit den Aktuatoren verbunden. Weiterhin ist die Steuereinrichtung 40 mit einem Schalter 45 verbunden, über den das Öffnen oder Schließen des Prothesenschaftes 1 eingeschaltet werden kann. Neben einer manuellen Betätigung und Aktivierung des Prothesenschaftes durch den Schalter 45 am proximalen Ende des frontalen Stützelementes 7 ist im distalen Bereich des Aufnahmeraumes ein Kontaktsensor 30 angeordnet, im dargestellten Ausführungsbeispiel im distalen Endbereich des Aufnahmeraums. Wird der Kontaktsensor 30 berührt, wird ein Signal an die Steuereinrichtung 40 gesendet, so dass automatisch eine Aktivierung des Aktuators/Motors oder Aktuatoren/Motoren erfolgt, um die Spanneinrichtungen 11, 12, 13 zu schließen. Der Kontaktsensor 30 kann auch als einfacher Schalter ausgebildet sein.

Entlang zumindest einem Stützelement 5 sind Drucksensoren 31, 32, 33 angeordnet, über die der Anpressdruck der Stützelemente 5, 6, 7, 8 überwacht wird. Werden voreingestellte Drücke erreicht, wird über die Steuereinrichtung 40 der jeweilige Aktuator deaktiviert. Vorteilhafterweise wird eine Druckverteilung eingestellt, wie sie in dem rechten Diagramm in Figur 1 gezeigt ist, bei der ein erhöhter Druck im distalen Bereich anliegt, der in Proximalrichtung abnimmt.

Weiterhin kann ein Inertialsensor 35 an dem Prothesenschaft 1 befestigt sein. Der Inertialsensor 35 erfasst die Lage des Prothesenschaftes 1 im Raum. Darüber können unterschiedliche Informationen gewonnen werden, die beispielsweise das Aktivitätsniveau betreffen, so dass in Abhängigkeit von der jeweils detektierten Aktivität eine Verstellung des Anpressdruckes durch Veränderung des Innenumfanges durch Öffnen oder Schließen der Spanneinrichtungen 11, 12, 13 erfolgen kann.

Neben dem dargestellten Inertialsensor 35 oder Inertialwinkelsensor, der die Orientierung zu einer festen Bezugsgröße, beispielsweise der Gravitationskraft, detektiert, können Lagesensoren, Beschleunigungssensoren oder andere Sensoren an dem Prothesenschaft 1 angeordnet und mit der Steuerungseinheit 40 gekoppelt sein, um darüber Informationen über die gegenwärtigen Aktivitäten zu erlangen. Ebenfalls kann eine Zeitschalteinrichtung in der Steuerungseinheit 40 abgelegt sein, über die eine zeitgesteuerte Anspannung oder Entspannung der Spanneinrichtungen 11, 12, 13 eingeleitet werden kann.

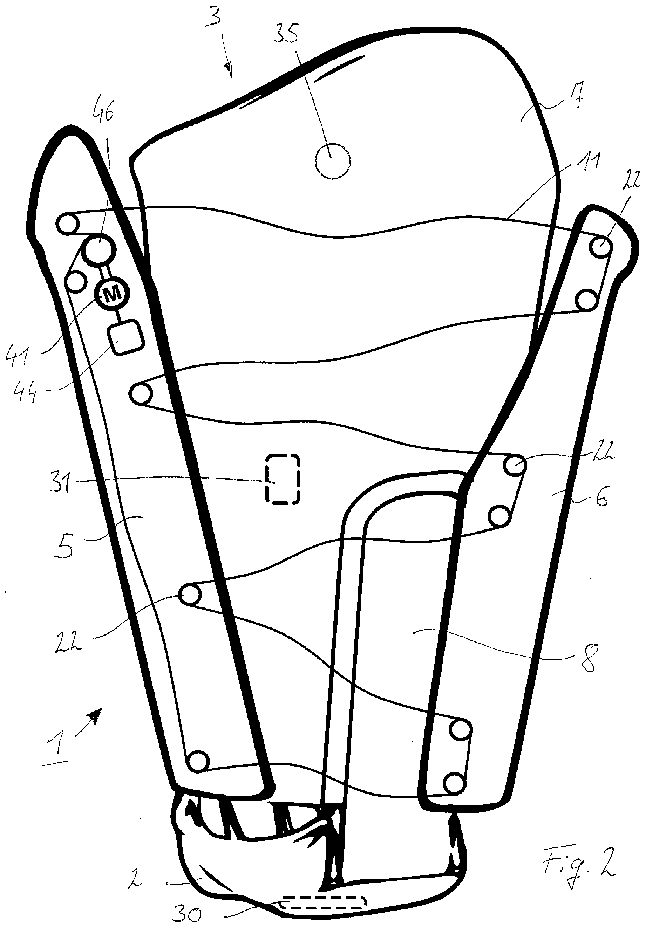

Figur 2 zeigt eine Variante der Erfindung in einer schematischen Darstellung im geöffneten Zustand. Gleiche Bezugszeichen bezeichnen gleiche Komponenten. Der Figur 2 ist zu entnehmen, dass die beiden seitlichen Stützelemente 5, 6 nach medial-lateral aufgeklappt sind, so dass sich eine aufgeweitete Einführöffnung 3 ergibt. Statt mehrerer separater Spanneinrichtungen 11, 12, 13 ist nur eine einzige Spanneinrichtung 11 in Gestalt eines Kabels oder einer Schnur an dem Außenumfang des Prothesenschaftes 1 angeordnet. Die ununterbrochene Spanneinrichtung 11 ist entlang von Umlenkrollen 22, die auf verschiedenen Ebenen oder Höhen in proximal-distal-Richtung angeordnet sind, entlang geführt. Die Spanneinrichtung 11 ist schlaufenartig mäandernd um die Umlenkrollen 22 herum geführt, wobei die Spanneinrichtung 11 auf einer Rolle 46 aufgerollt wird, wenn ein Aktuator 41 in Gestalt eines Motors in einer entsprechenden Drehrichtung angetrieben wird. Dem Motor 41 ist ein Motorstromsensor 44 zugeordnet, der den aufgebrachten Motorstrom misst. Über den Motorstrom ist es möglich, das Motormoment und damit die Kraft, mit welcher die Spanneinrichtung 11 gespannt wird, zu detektieren. Über die Spannkraft der Spanneinrichtung 11 kann auf den aufgebrachten Anpressdruck der Stützelemente 5, 6, 7, 8 auf den Stumpf geschlossen werden.

Neben dem Motorstromsensor 44 ist an der Innenseite des frontalen Stützelementes 7 ein Drucksensor 31 angeordnet, um einen weiteren Messwert der nicht dargestellten Steuereinheit 40 übermitteln zu können.

Nicht dargestellt ist ein rückwärtiges Stützelement, das ebenfalls überlappend mit den medialen und lateralen Stützelementen 5, 6 an dem Endstück 2 angeordnet ist, so dass sich eine vollständige Umschließung des nicht dargestellten Stumpfes ergibt.

An dem Boden des Aufnahmeraumes an dem Endstück 2 ist der distale Kontaktsensor 30 angeordnet. Die Stützelemente 5, 6, 7, 8 sind nach außen gerichtet vorgespannt oder elastisch ausgebildet, so dass nach Entspannung der Spanneinrichtung 11 durch Abwickeln der Spanneinrichtung 11 von der Rolle 46 der Prothesenschaft 1 sich öffnet.

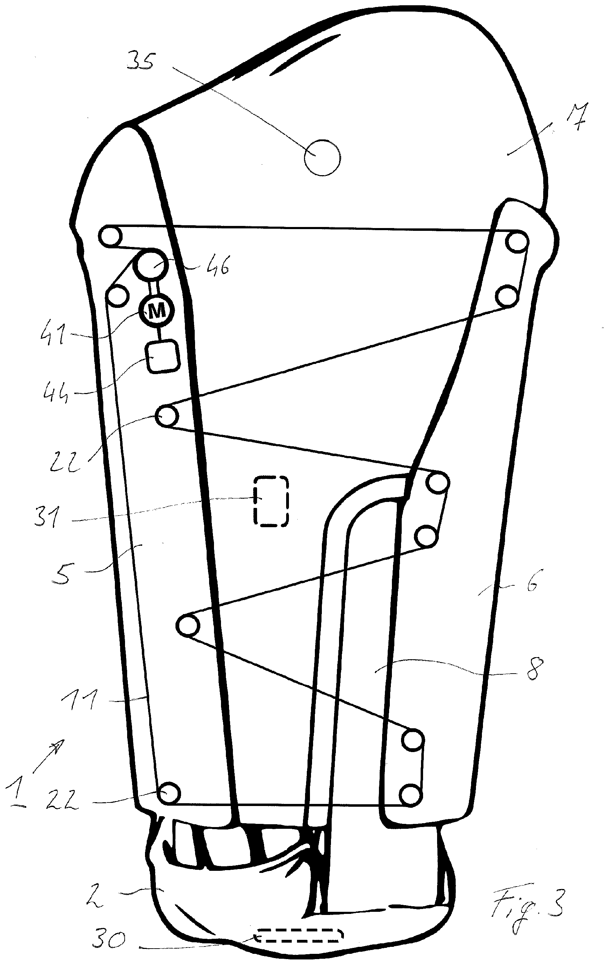

Figur 3 zeigt den Prothesenschaft 1 gemäß Figur 2 in einem geschlossenen Zustand. Ein nicht dargestellter Stumpf ist durch die Einführöffnung 3 in den Aufnahmeraum eingeführt worden und hat den Kontaktsensor 30 aktiviert. Aufgrund des Aktivierungssignals der nicht dargestellten Steuerungseinheit 40 wurde der Motor 41 in Betrieb gesetzt. Die Spanneinrichtung 11 wurde auf die Rolle 46 aufgewickelt, bis der Motorstromsensor 44 oder der Drucksensor 31 ein Signal an die Steuereinheit 40 übermittelt, bis der gewünschte oder voreingestellte Grenzdruck erreicht wird. Die Steuereinheit 40 hat den Motor 41 ausgeschaltet und hält die Spanneinrichtung 11 im gespannten Zustand. Die Spanneinrichtung 11 ist senkrecht entlang des medialen Stützelementes 4 in proximal-distal-Richtung geführt, quert frontal im unteren Bereich der Stützelemente 5, 6, 7, 8 und wird durch Umlenkrollen 22 umgelenkt, so dass sich eine zick-zack-artige Anordnung oder ein zick-zack-artiger Verlauf zwischen dem medialen Stützelement 5 und dem lateralen Stützelement 6 ergibt. Im proximalen Endbereich quert die Spanneinrichtung 11 wieder frontal und ist mit der Rolle 46 gekoppelt und wird dort aufgewickelt. Neben einer permanenten Beaufschlagung des Motors 41 mit einem Haltestrom ist es möglich und vorgesehen, dass eine mechanische Verriegelungseinrichtung der Spanneinrichtung 11 zugeordnet ist, so dass der Motor 41 stromlos geschaltet werden kann. Zur Entriegelung kann dann die mechanische Verriegelungseinrichtung entsperrt und der Motor 41 in eine umgekehrte Drehrichtung aktiviert werden, um den Prothesenschaft 1 zu öffnen.

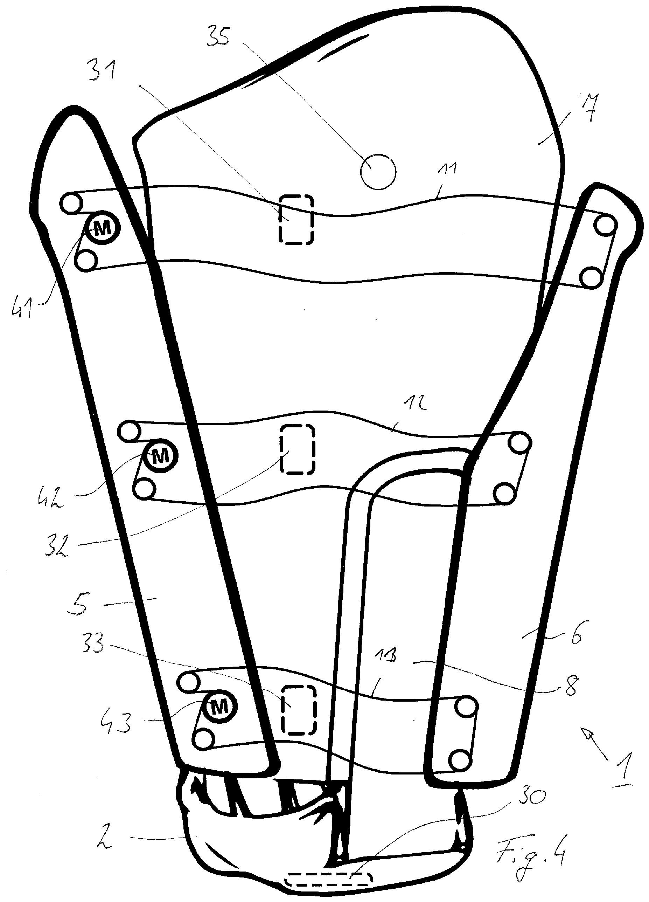

Eine Variante der Ausführungsform gemäß Figur 3 ist in der Figur 4 gezeigt. Statt nur eines einzigen Motors 41 sind drei Motoren 41, 43 in unterschiedlichen Höhen in proximal-distal-Richtung an dem Prothesenschaft 1 festgelegt. Jeder Motor 1 ist einer Spanneinrichtung 11, 12, 13 zugeordnet. Auf den jeweiligen Höhen der Motoren 41, 42, 43 sind Drucksensoren 31, 32, 33 an der Innenseite des Prothesenschaftes 1 im Aufnahmeraum angeordnet, im dargestellten Ausführungsbeispiel an dem frontalen Stützelement 7. Die Drucksensoren 31, 32, 33 können auch an einem anderen Stützelement, 5, 6, 7, 8 angeordnet sein, ebenso können mehrere Sensoren 31, 32, 33 an jedem Stützelement 5, 6, 7 8, ebenso können Sensoren in unterschiedlichen Höhen an verschiedenen Stützelementen angeordnet sein.

Ebenso ist es möglich, dass zusätzlich zu mehreren Sensoren mehrere Motorstromsensoren den jeweiligen Motoren 41, 42, 43 zugeordnet sind.

Alle Motoren 41, 42, 43 sind mit der nicht dargestellten Steuereinheit 40 verbunden. Wird der Sensor 30 oder Kontaktschalter 30 aktiviert, werden die Motoren 41, 42, 43 dergestalt aktiviert, dass die Spanneinrichtungen 11, 12, 13 aufgewickelt werden, beispielsweise auf eine Motorwelle.

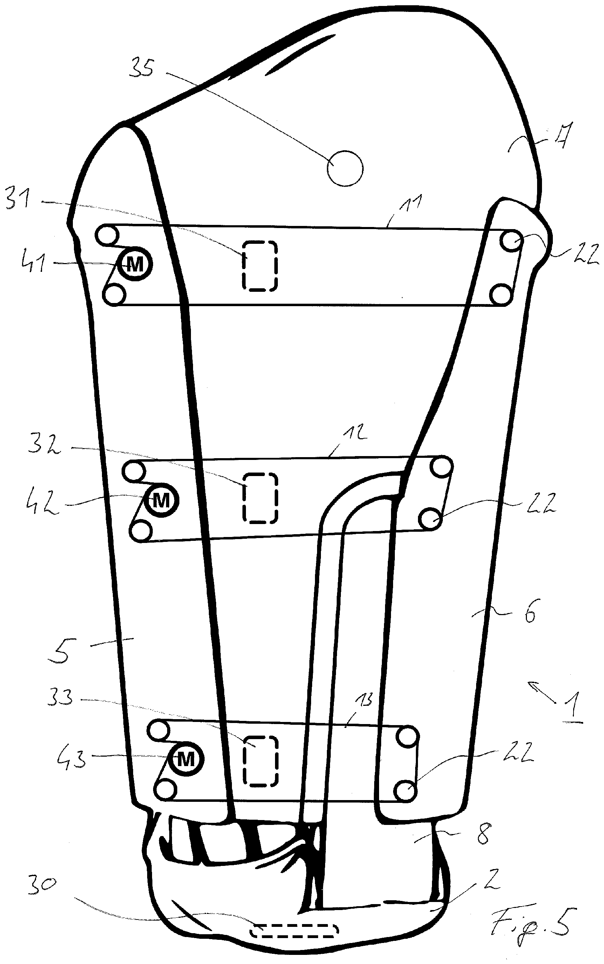

In Figur 5 ist der geschlossene Zustand dargestellt, nachdem die Motoren 41, 42, 43 die Spanneinrichtungen 11, 12, 13 gespannt haben. Die Stützelemente 5, 6, 7, 8 sind aufeinander zu bewegt worden, bis der jeweilige Sensor 31, 32, 33 den vorgegebenen Anpressdruck angezeigt hat. Dann werden die Motoren 41, 42, 43 entsprechend abgeschaltet, der Prothesenschaft 1 ist geschlossen.

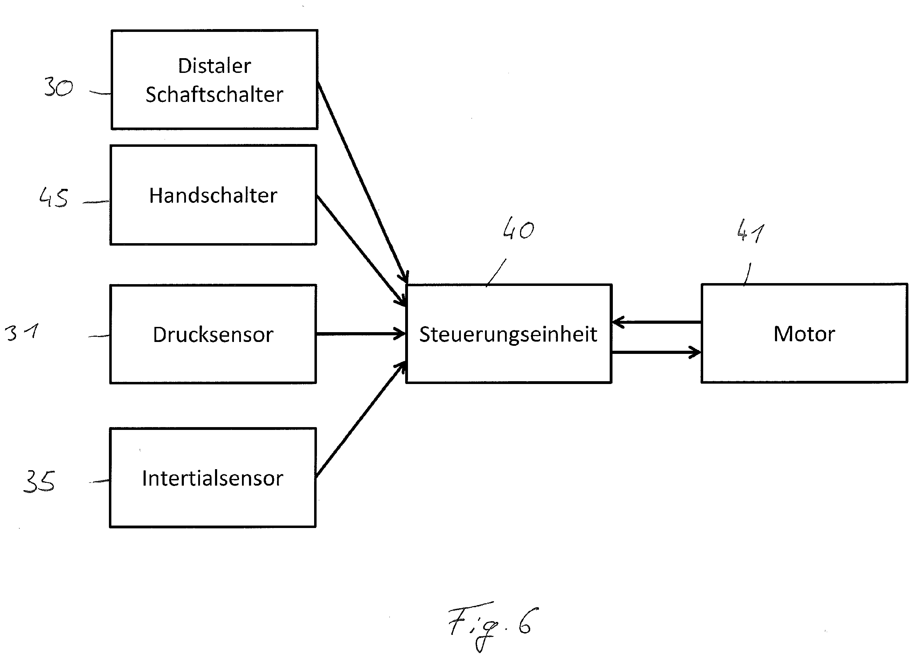

Figur 6 zeigt eine Schemadarstellung der Motorsteuerung. Die Steuerungseinheit 40 ist mit dem Motor 41 oder den Motoren verbunden. Ebenfalls mit der Steuerungseinheit 40 ist der distale Schaftschalter 30, der manuelle Schalter 45, der oder die Drucksensoren 31 sowie der Inertialsensor 35 verbunden. Weitere Sensoren oder Schalter können ebenfalls mit der Steuerungseinheit 40 verbunden sein. Auf der Grundlage der Informationen durch die Sensoren oder Befehle durch die Schalter aktiviert die Steuerungseinheit 40 den Motor und spannt oder entspannt die jeweils zugeordnete Spanneinrichtung.

Der erfindungsgemäße Prothesenschaft ermöglicht ein selbsttätiges Öffnen und Schließen durch entsprechende Aktivierung oder Deaktivierung eines Aktuators oder mehrerer Aktuatoren, die mit einer oder mehreren Spanneinrichtungen, beispielsweise einem Schnürsystem oder mehreren Schnürsystemen verbunden sind. Statt der Betätigung von Zugmitteln, die aufgerollt und abgerollt werden, können die Aktuatoren auch andere Wirkelemente wie Hebel, Zahnräder und Zahnstangen, Schieber, Spindeln, Spreizkeile, Ringe oder Spangen betätigen und verlagern, um eine Innenumfangsveränderung auf Grundlage von Sensordaten zu bewirken. Neben einer mehrteiligen Ausgestaltung des Prothesenschaftes mit mehreren Stützelementen, die zueinander verlagerbar sind, kann der Prothesenschaft auch mit nur einer einteiligen Schaftwand ausgebildet sein, die einen Schlitz aufweist oder ähnlich einer Fackel spiralartig aufgewickelt ist. Bei einer konischen Ausgestaltung kann ein Öffnen oder Schließen des Prothesenschaftes durch Verschieben eines Ringes oder einer Spange entlang der Längserstreckung des Schaftes erfolgen. Andere Maßnahmen zur Veränderung des Innenumfanges wurden bereits oben beschrieben. Der Prothesenschaft hat unter anderem den Vorteil, dass auch bei Schwankungen im Stumpfvolumen oder bei unterschiedlichen Stümpfen eine korrekte Anpassung des Prothesenschaftes an den Stumpf erfolgen kann. Es ist somit nicht mehr notwendig, einen individuell abgeformten Prothesenstumpf bereitzustellen. In dem Grundzustand ist der Prothesenschaft geöffnet, das heißt, dass die Stützelemente aufgetulpt sind. Wird der distale Schaftschalter aktiviert, wobei der distale Schalter als Kontaktschalter, Druckschalter, mechanischer Schalter oder Näherungsschalter ausgebildet sein kann, wird der Aktuator oder werden die Aktuatoren aktiviert, bis der voreingestellte Normalanpressdruck der Schaftwand oder der Stützelemente an den Stumpf erreicht wird. In diesem Normalmodus sind die üblichen Tätigkeiten ausführbar, beispielsweise bei einer Prothese der unteren Extremität Gehen oder Treppensteigen.

Werden hohe Beschleunigungen über einen bestimmten Zeitraum detektiert, beispielsweise durch einen an dem Prothesenschaft oder der Prothese angeordneten Beschleunigungssensor, wird über die Steuerungseinheit einer oder mehrere Aktuatoren aktiviert, um den Innenumfang zu verringern und ggf. die Spanneinrichtung oder die Spanneinrichtungen nachzuspannen. Der Änderungs- oder Spannvorgang wird solange fortgesetzt, bis ein voreingestellter Anpressdruck erreicht wird, der für den detektierten Belastungsmodus, beispielsweise beim Sport geeignet erscheint.

Sobald keine erhöhten Beschleunigungen oder Kräfte mehr detektiert werden und das Nichtvorliegen besondere Belastungen über einen bestimmten Zeitraum festgestellt wird, wird der Anpressdruck reduziert, indem die Spanneinrichtungen gelöst, Volumina verringert, Magnet aktiviert oder deaktiviert, Formgedächnislegierungen aktiviert oder deaktiviert oder andere Veränderungen vorgenommen werden, bis der Normaldruck wieder erreicht wird. Der Prothesenschaft öffnet sich aufgrund von den Stützelementen oder der Schaftwand innewohnenden Elastizitäten oder einer separaten Rückstelleinrichtung, beispielweise eine Feder, die den Prothesenschaft von dem Stumpf weg mit einer Kraft belasten.

Werden wiederum besondere geringe Belastungen detektiert oder aber auch Anpressdruckerhöhungen, die nicht durch erhöhte Beschleunigungswerte innerhalb der Prothese oder des Prothesenschaftes verursacht sind, beispielsweise aufgrund von Volumenzunahmen innerhalb des Stumpfes, kann der Aktuator oder können die Aktuatoren ausgehend von dem Normalmodus die Spanneinrichtungen oder andere Mittel zur Änderung des Innenumfanges entspannen, so dass sich der Prothesenschaft in einem Entspannungsmodus mit einem niedrigeren Anpressdruck aufgrund des vergrößerten Innenumfanges befindet. Dies kann beispielsweise im Sitzen oder im Liegen geschehen. Solche Zustände können über Beschleunigungssensoren, Lagesensoren und Inertialsensoren detektiert werden. Wird eine Bewegungsaufnahme detektiert, beispielsweise über Beschleunigungssensoren, spannen die Motoren oder spannt der Motor die Spanneinrichtung oder die Spanneinrichtungen wieder an oder verändert die Pumpe das Volumen in dem aufblasbaren Polster, bis der Normaldruck erreicht wird. Die Anpressdrücke können wieder direkt über Drucksensoren oder indirekt über Motorstromsensoren oder andere Sensoren ermittelt werden.

Zum Öffnen des Prothesenschaftes wird ggf. ein manueller Schalter betätigt, der die Spanneinrichtung oder die Spanneinrichtungen maximal entspannt oder den Aktuator zu einer maximalen Innenumfangsvergrößerung aktiver. Falls die Aktuatoren/Motoren oder der Aktuator/Motor ausfallen sollten oder sollte, kann eine mechanische Entsperreinrichtung vorgesehen sein, über die die Spanneinrichtung vollständig entspannt oder der Innenumfang vergrößert werden kann, um einen Ausstieg aus dem Prothesenschaft zu ermöglichen.

Vorteilhafterweise wird der Prothesenschaft geschlossen, sobald über den distalen Schaftsensor oder Schaftschalter angezeigt wird, dass der Stumpf oder die Gliedmaße vollständig eingeführt worden ist. Alternativ oder ergänzend zu einer automatischen Aktivierung der Schließeinrichtung kann der Schaft über einen separaten Schließknopf, einen Magnetschalter, ein Smartphone, ein Tablet, eine Fernbedienung oder dergleichen geschlossen werden, dazu ist die Steuerungseinheit mit einem Empfängermodul oder einem entsprechenden Kontakt mit dem Schalter verbunden. Eine Sprachsteuerung kann ebenfalls vorgesehen sein, um den Prothesenschaft zu öffnen oder zu schließen.

Durch wechselseitiges Spannen und Entspannen der Spanneinrichtungen kann eine Massagewirkung für den aufgenommenen Stumpf bereitgestellt werden, was für den Prothesennutzer angenehm sein kann.

Neben einer Anpassung auf der Grundlage von Aktivitäten kann ein Anspannen oder Entspannen der Spanneinrichtung im Rahmen eines Zeitprogrammes erfolgen, um unerwünschte Druckstellen und Schädigungen des Stumpfgewebes zu vermeiden. Die Sensoren zur Erfassung der jeweiligen Aktivitäten können sowohl an dem Prothesenschaft als auch an der angeschlossenen Protheseneinrichtung angeordnet sein, die dort befindlichen Sensoren, beispielsweise Inertialwinkelsensoren, Beschleunigungssensoren, Gyroskope oder Winkelsensoren können genutzt werden, um eine angepasste Veränderung des Innenumfanges, z.B. durch eine Anspannung oder Entspannung von Spanneinrichtungen zu bewirken.

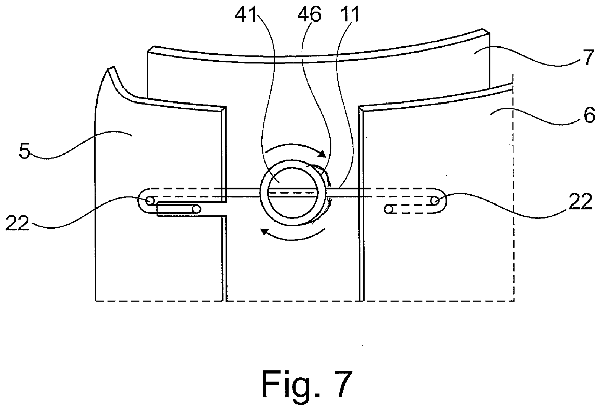

Figur 7 zeigt in einer Detailansicht eine schematische Darstellung eines Prothesenschaftes 1 mit drei Stützelementen 5, 6, 7, von denen jeweils zwei einander überlappend angeordnet sind. Die Stützelemente 5, 6 sind so angeordnet, dass sie einen Innenumfang des Prothesenschaftes ausbilden, der kleiner als der Außenumfang eines aufzunehmenden Stumpfes oder eines aufzunehmenden Stumpfes mit Prothesenliner ist. Dies kann durch eine entsprechende Vorformung oder aber durch Vorspannkraft bewirkt werden, die eine Verlagerung der Stützelemente 5, 6 in Richtung auf dem Stumpf bewirkt. Dies kann beispielsweise eine Federkraft sein, die die gelenkig an einem Endstück gelagerten Stützelemente 5, 6 in Richtung auf den nicht dargestellten Stumpf verlagert oder vorspannt. An dem dritten Stützelement 7 ist ein Aktuator 41 in Gestalt eines Motors angeordnet, der mit einer Rolle 46 gekoppelt ist, an der ein Spannelement 11 in Gestalt eines Kabels befestigt ist. Ebenfalls ist an dem Stützelement 7, an dem der Aktuator 41 befestigt ist, ein Paar Umlenkelemente 22 angeordnet, die auf einander gegenüberliegenden Seiten der Rolle 46 positioniert sind. Das Kabel 11 ist an der Rolle 46 befestigt und um die Umlenkelemente oder Umlenkrollen 52 herum geführt, wobei die freien, nicht an der Rolle 46 befestigten Enden des Kabels 11 wieder zurück in Richtung auf die Rolle 46 geführt sind. Die Führung des Kabels 11 ist somit U-förmig. Jeweils ein freies Ende ist an dem Stützelement 5, 6 befestigt, das die jeweilige Umlenkrolle 22 überdeckt. Sofern der Prothesenschaft nur aus zwei Stützelementen besteht, also zwischen einer Schaftwand ein Spalt oder ein Schlitz ausgebildet ist, der von dem zweiten Stützelement 7 überdeckt wird, sind die freien Enden jeweils im Bereich der einander gegenüberliegenden Kanten beiderseits der Rolle 46 festgelegt.

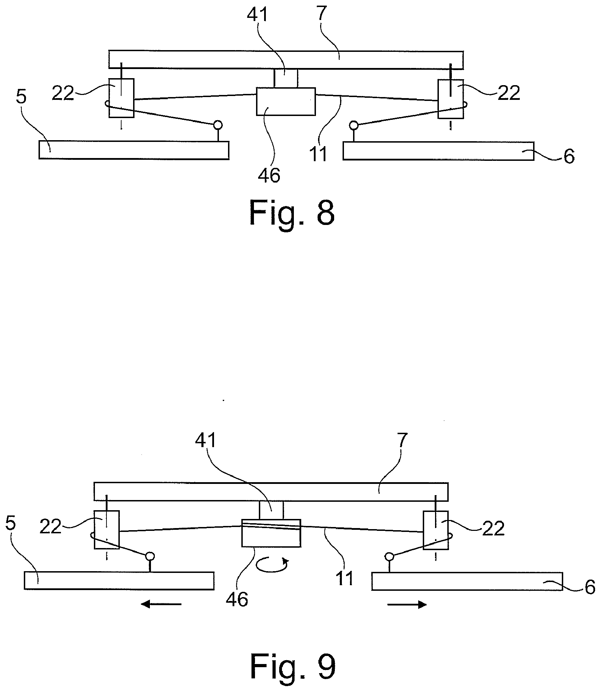

Figur 8 zeigt den Aufbau der Spreizeinrichtung gemäß Figur 7 im nicht geöffneten Zustand. Das Kabel 11 ist in entgegengesetzten Richtungen um die beiden zueinander beabstandet angeordneten Umlenkrollen 22 und wieder zurück zu den Kanten der beiden verlagerbaren Stützelemente 5, 6 geführt. Dort sind die freien Enden des Kabels 11 an jeweils einer Befestigungsstelle befestigt. Das Kabel 11 ist maximal abgerollt, das bedeutet, dass die Stützelemente 5, 6 maximal aufeinander zu bewegt sind und sich dadurch ein minimaler Innenumfang des Prothesenschaftes ergibt. Soll nun der Innenumfang vergrößert werden, wird der Aktuator 41 in Gestalt eines Motors aktiviert, was in der Figur 9 dargestellt ist. Durch das Aktivieren des Aktuators 41 wird die Rolle 46 in Drehung versetzt, das Kabel 11 wird aufgerollt und die Befestigungsstellen mit den freien Enden des Kabels 11 werden in Richtung auf die jeweilige Umlenkrolle 22 gezogen. Dadurch vergrößert sich der Spalt oder Abstand zwischen den beiden Stützelementen 5, 6 oder der Spalt in einer Prothesenschaftwand, wodurch sich der Innenumfang vergrößert und ein Einsteigen in den Prothesenschaft erleichtert oder ein Aussteigen erleichtert oder ermöglicht wird. Zum Schließen des Prothesenschaftes 1 wird eine umgekehrte Bewegung durch den Aktuator 41 veranlasst, das Kabel 11 wird abgerollt und die beiden Stützelemente 5, 6 bewegen sich entgegen der Pfeilrichtung gemäß Figur 9 wieder aufeinander zu. Dadurch wird der Innenumfang verringert, der Druck auf den innerhalb des Prothesenschaftes angeordneten Stumpf wird vergrößert und eine verbesserte Passform wird erreicht.

Grundsätzlich ist es möglich und vorgesehen, dass die oben beschriebenen Ausführungsbeispiele auch ohne Sensoren und ohne Steuerungseinrichtung funktionieren und betrieben werden, wobei der Aktuator 41 dann entweder motorisch oder manuell betätigt wird. Insbesondere das manuelle oder motorische Öffnen ohne Sensoren und Steuereinrichtung durch eine Aktivierung eines Motors oder durch manuelle Betätigung eines Aktuators, beispielsweise einer Zahnstange, eines Schiebers, eines Gewindes, einer Rolle, einer Verstellung eines Spreizelementes, beispielsweise eines Ringes, einer Spange oder dergleichen, ermöglicht es, den Innenumfang zu vergrößern. Die Rückstellung erfolgt vorteilhafterweise durch eine elastische Rückstellkraft, die dem geöffneten Prothesenschaft aufgeprägt wird. Eine Ausgestaltung ohne Sensor stellt eine eigenständige Lösung und Erfindung dar.

## Patentansprüche

1. Prothesenschaft mit
a. einer proximalen Einführöffnung (3) und einem einen Stumpf zumindest teilweise umgebenen Innenumfang,
b. zumindest einer Anschlussvorrichtung (4) für eine Prothesenkomponente, die an dem Prothesenschaft befestigbar ist,
c. und zumindest einem Aktuator (41), über den der Innenumfang des Prothesenschaftes (1) vergrößerbar ist,
**dadurch gekennzeichnet, dass** der Prothesenschaft (1) über eine elastische Rückstellkraft an dem Stumpf hält und der Aktuator (41) ausgebildet ist, den Innenumfang des Prothesenschaftes (1) entgegen der Rückstellkraft zu vergrößern.

2. Prothesenschaft nach Anspruch 1, **dadurch gekennzeichnet, dass** der Aktuator (41) manuell oder motorisch antreibbar ist.

3. Prothesenschaft nach einem der voranstehenden Ansprüche, **dadurch gekennzeichnet, dass** der Prothesenschaft (1) mehrteilig mit zumindest zwei Stützelementen (5, 6, 7, 8) ausgebildet ist, die den Stumpf zwischen sich aufnehmen.

4. Prothesenschaft Anspruch 3, **dadurch gekennzeichnet, dass** die Stützelemente (5, 6, 7, 8) elastisch ausgebildet oder gelagert sind.

5. Prothesenschaft nach Anspruch 3 oder 4, **dadurch gekennzeichnet, dass** die Stützelemente (5, 6, 7, 8) in Umfangrichtung einander überlappend ausgebildet sind.

6. Prothesenschaft nach einem der voranstehenden Ansprüche, **dadurch gekennzeichnet, dass** an dem Prothesenschaft (1) ein durch den Aktuator (41) veränderbares Volumen angeordnet oder ausgebildet ist, über das der Innenumfang veränderbar ist.

7. Prothesenschaft nach einem der voranstehenden Ansprüche, **dadurch gekennzeichnet, dass** zumindest eine Spanneinrichtung (11, 12, 13) an dem Prothesenschaft (1) angeordnet ist, um dessen Innenumfang zu verändern.

8. Prothesenschaft nach Anspruch 7, **dadurch gekennzeichnet, dass** die Spanneinrichtung (11, 12, 13) als Zugmittel, pneumatisch oder hydraulisch angetriebenes Spreiz- oder Spannmittel, als längsverschiebliches Spreiz- oder Schließelement, als verfahrbarer Ring, als Kippelement oder verdrehbares Betätigungselement ausgebildet ist

9. Prothesenschaft nach Anspruch 7 oder 8, **dadurch gekennzeichnet, dass** die Spanneinrichtung (11, 12, 13) in Ösen (21), Kanälen und/oder um zumindest eine Umlenkrolle (22) geführt ist.

10. Prothesenschaft nach einem der Ansprüche 7 bis 9, **dadurch gekennzeichnet, dass** mehrere Spanneinrichtungen (11, 12, 13) unabhängig voneinander entlang der proximal-distal-Erstreckung des Prothesenschaftes (1) angeordnet sind.

11. Prothesenschaft nach einem der Ansprüche 7 bis 10, **dadurch gekennzeichnet, dass** die zumindest eine Spanneinrichtung (11, 12, 13) in Umfangsrichtung des Stumpfes wirkt.

12. Prothesenschaft nach einem der voranstehenden Ansprüche, **dadurch gekennzeichnet, dass** im geschlossenen Zustand der auf den Stumpf aufgebrachte Anpressdruck (p) in Proximalrichtung abnimmt.

13. Verfahren zur Anpassung eines Innenumfanges eines Prothesenschaftes, **dadurch gekennzeichnet, dass** zum Öffnen des Prothesenschaftes (1) Stützelemente (5, 6, 7, 8) durch einen Aktuator (41) voneinander weg verlagert, eine Schaftwand auseinandergezogen oder zur Umfangsveränderung eine gegen eine elastische Vorspannkraft der Stützelemente (5, 6, 7, 8) oder der Schaftwand wirkende Spanneinrichtung (11, 12, 13) entspannt werden.

## Claims

1. A prosthesis socket with:
a. a proximal insertion opening (3) and an inner circumference which at least partially surrounds a limb stump,
b. at least one connection device (4) for a prosthesis component, which is connectable to the prosthesis socket,
c. and at least one actuator (4), by means of which the inner circumference of the prosthesis socket (1) is enlargeable, **characterized in that** the prosthesis socket (1) is held by a resilient reset force on the limb stump and the actuator (41) is designed to increase the inner circumference of the prosthesis socket (1) against the resilient reset force.

2. The prosthesis socket according to Claim 1, **characterized in that** the actuator (41) can be driven manually or by a motor.

3. The prosthesis socket according to any one of the preceding claims, **characterized in that** the prosthesis socket (1) is designed in multiple parts with at least two support elements (5, 6, 7, 8) which receive the limb stump in between themselves.

4. The prosthesis socket according to Claim 3, **characterized in that** the support elements (5, 6, 7, 8) are formed or mounted resiliently.

5. The prosthesis socket according to Claim 3 or 4, **characterized in that** the support elements (5, 6, 7, 8) are designed to overlap one another in circumferential direction.

6. The prosthesis socket according to any one of the preceding claims, **characterized in that**, on the prosthesis socket (1), a volume which is changeable by the actuator (41) is arranged or formed, by means of which the inner circumference can be changed.

7. The prosthesis socket according to any one of the preceding claims, **characterized in that** at least one tensioning device (11, 12, 13) is arranged on the prosthesis socket (1), in order to change its inner circumference.

8. The prosthesis socket according to Claim 7, **characterized in that** the tensioning device (11, 12, 13) is designed as a traction means, a pneumatically or hydraulically driven spreading or tensioning means, as a longitudinally slidable spreading or closing element, as a movable ring, as a tilting element or a rotatable actuation element.

9. The prosthesis socket according to Claim 7 or 8, **characterized in that** the tensioning device (11, 12, 13) is guided in eyelets (21), channels and/or around at least one deflection roller (22).

10. The prosthesis socket according to any one of Claims 7 to 9, **characterized in that** multiple tensioning devices (11, 12, 13) are arranged independently of one another along the proximal-distal extent of the prosthesis socket (1).

11. The prosthesis socket according to any one of Claims 7 to 10, **characterized in that** the at least one tensioning device (11, 12, 13) acts in circumferential direction of the limb stump.

12. The prosthesis socket according to any one of the preceding claims, **characterized in that**, in the closed state, the contact pressure (p) applied to the limb stump decreases in proximal direction.

13. A method for adjusting an inner circumference of a prosthesis socket, **characterized in that**, for the opening of the prosthesis socket (1), support elements (5, 6, 7, 8) are moved apart from one another by an actuator (41), a socket wall is pulled apart, or, for the change in circumference, a tensioning device (11, 12, 13) acting against a resilient pretensioning force of the support elements (5, 6, 7, 8) or of the socket wall is relaxed.

## Revendications

1. Emboîture de prothèse présentant
a. une ouverture d'insertion proximale (3) et un pourtour intérieur entourant au moins partiellement un moignon,
b. au moins un dispositif de raccordement (4) pour un composant prothétique, qui peut être fixé à l'emboîture de prothèse,
c. et au moins un actionneur (41) au moyen duquel le pourtour intérieur de l'emboîture de prothèse (1) peut être agrandi,
**caractérisée en ce que** l'emboîture de prothèse (1) est maintenue contre le moignon par une force de rappel élastique, et l'actionneur (41) est réalisé pour augmenter le pourtour intérieur de l'emboîture de prothèse (1) à l'encontre de la force de rappel.

2. Emboîture de prothèse selon la revendication 1, **caractérisée en ce que** l'actionneur (41) peut être entraîné manuellement ou par voie motrice.

3. Emboîture de prothèse selon l'une des revendications précédentes, **caractérisée en ce que** l'emboîture de prothèse (1) est réalisée en plusieurs parties avec au moins deux éléments de soutien (5, 6, 7, 8) qui reçoivent le moignon entre eux.

4. Emboîture de prothèse selon la revendication 3, **caractérisée en ce que** les éléments de soutien (5, 6, 7, 8) sont réalisés ou montés de manière élastique.

5. Emboîture de prothèse selon la revendication 3 ou 4, **caractérisée en ce que** les éléments de soutien (5, 6, 7, 8) sont réalisés de manière à se chevaucher dans la direction circonférentielle.

6. Emboîture de prothèse selon l'une des revendications précédentes, **caractérisée en ce qu'**un volume qui peut être modifié par l'actionneur (41) est disposé ou réalisé sur l'emboîture de prothèse (1), par lequel le pourtour intérieur peut être modifié.

7. Emboîture de prothèse selon l'une des revendications précédentes, **caractérisée en ce qu'**au moins un dispositif tendeur (11, 12, 13) est disposé sur l'emboîture de prothèse (1) afin de modifier son pourtour intérieur.

8. Emboîture de prothèse selon la revendication 7, **caractérisée en ce que** le dispositif tendeur (11, 12, 13) est réalisé sous forme de moyen de traction, de moyen d'écartement ou de tensionnement à commande pneumatique ou hydraulique, d'élément d'écartement ou de fermeture coulissant longitudinalement, d'anneau déplaçable, d'élément de basculement ou sous forme d'élément d'actionnement rotatif.

9. Emboîture de prothèse selon la revendication 7 ou 8, **caractérisée en ce que** le dispositif tendeur (11, 12, 13) est guidé dans des œillets (21), dans des canaux et/ou autour d'au moins un galet de renvoi (22).

10. Emboîture de prothèse selon l'une des revendications 7 à 9, **caractérisée en ce que** plusieurs dispositifs tendeurs (11, 12, 13) sont disposés indépendamment les uns des autres le long de l'extension proximale-distale de l'emboîture de prothèse (1).

11. Emboîture de prothèse selon l'une des revendications 7 à 10, **caractérisée en ce que** ledit au moins un dispositif tendeur (11, 12, 13) agit dans la direction circonférentielle du moignon.

12. Emboîture de prothèse selon l'une des revendications précédentes, **caractérisée en ce qu'**à l'état fermé, la pression de pressage (p) appliquée au moignon diminue en direction proximale.

13. Procédé d'adaptation d'un pourtour intérieur d'une emboîture de prothèse, **caractérisé en ce que**, pour ouvrir l'emboîture de prothèse (1), des éléments de soutien (5, 6, 7, 8) sont déplacés en éloignement les uns des autres par un actionneur (41), une paroi d'emboîture est étirée, ou, pour modifier le pourtour, un dispositif tendeur (11, 12, 13) agissant à l'encontre d'une force de précontrainte élastique des éléments de soutien (5, 6, 7, 8) ou de la paroi d'emboîture est détendu.
